# FASCICULE DE BREVET EUROPEEN

(11) **EP 3 606 903 B1**
(45) Date de publication et mention de la délivrance du brevet: **12.07.2023**
(21) Numéro de dépôt: 18718888.3
(22) Date de dépôt: 03.04.2018
(51) Int. Cl.: C07C 51/44, C07C 57/04

(54) **PROCEDE DE PURIFICATION D'ACIDE (METH)ACRYLIQUE INCLUANT UNE COLONNE DE DISTILLATION A PAROI SEPARATRICE.**
VERFAHREN ZUR REINIGUNG VON (METH)ACRYLSÄURE MIT EINER TRENNWAND-DESTILLATIONSKOLONNE
PROCESS FOR PURIFYING (METH)ACRYLIC ACID INCLUDING A DIVIDING-WALL DISTILLATION COLUMN

(30) Priorité: 04.04.2017 FR 1752902
(43) Date de publication de la demande: 12.02.2020
(73) Titulaire: Arkema France, 92700 Colombes (FR)
(72) Inventeur: FAUCONET, Michel, 57730 Valmont (FR); TRETJAK, Serge, 57520 Roulhing (FR)
(74) Mandataire: Arkema Patent
(86) Numéro de dépôt international: PCT/FR2018/050826
(87) Numéro de publication internationale: WO 2018/185423

(56) Documents cités:
- EP-A2- 2 066 613

## Description

### DOMAINE TECHNIQUE

La présente invention concerne la production d'acide (méth)acrylique.

Elle a plus particulièrement pour objet la mise en oeuvre d'une colonne à paroi séparatrice comme colonne de purification/finition dans un procédé de récupération d'acide (méth)acrylique basé sur la mise en oeuvre de deux colonnes de distillation en l'absence de solvant organique externe. Le procédé selon l'invention permet d'améliorer le bilan énergétique du procédé tout en améliorant la qualité technique de l'acide (méth)acrylique récupéré.

Le procédé selon l'invention permet en outre de produire directement de l'acide (méth)acrylique de grade polymère (ou glacial) compatible avec la fabrication de polymères d'acide acrylique de hautes masses moléculaires.

### ARRIERE-PLAN TECHNIQUE ET PROBLEME TECHNIQUE

Le procédé de synthèse d'acide acrylique exploité à grande échelle industrielle, met en oeuvre une réaction d'oxydation catalytique du propylène en présence d'oxygène.

Cette réaction est conduite généralement en phase gazeuse, et le plus souvent en deux étapes : la première étape réalise l'oxydation sensiblement quantitative du propylène en un mélange riche en acroléine, puis, lors de la deuxième étape réalise l'oxydation sélective de l'acroléine en acide acrylique.

Le mélange gazeux issu de la deuxième étape est constitué, en dehors de l'acide acrylique, de composés non transformés issus des réactifs mis en jeu ou d'impuretés générées lors de l'une au moins des 2 étapes de réaction, à savoir
- de composés légers incondensables dans les conditions de température et de pression habituellement mises en oeuvre, soit essentiellement : propylène, propane, azote, oxygène non converti, monoxyde et dioxyde de carbone formés en faible quantité par oxydation ultime;
- de composés légers condensables, soit essentiellement : eau, des aldéhydes légers comme acroléine non converti, formaldéhyde, glyoxal et acétaldéhyde, acide formique, acide acétique, acide propionique ;
- de composés lourds : furfuraldéhyde, benzaldéhyde, acide et anhydride maléique, acide benzoïque, acide 2-buténoïque, phénol, protoanémonine.

La complexité du mélange gazeux obtenu dans ce procédé nécessite de procéder à un ensemble d'opérations pour récupérer l'acide acrylique contenu dans cet effluent gazeux et le transformer en un grade d'acide acrylique compatible avec son utilisation finale, par exemple la synthèse d'esters acryliques ou la production de polymères d'acide acrylique et/ou d'esters acryliques.

Une nouvelle technologie de récupération/purification d'acide acrylique est apparue récemment, impliquant un nombre réduit d'étapes de purification et ne nécessitant pas de solvant organique externe.

Le brevet EP 2 066 613, basé sur cette technologie « sans solvant », décrit un procédé de récupération d'acide acrylique sans utiliser d'eau extérieure, ni de solvant azéotropique. Ce procédé ne met en oeuvre que deux colonnes de distillation pour purifier le mélange réactionnel gazeux refroidi : a) une colonne de déshydratation, b) et une colonne de finition (ou colonne de purification) alimentée par une partie du flux de pied de la colonne de déshydratation.

Selon ce procédé, le flux réactionnel gazeux refroidi est soumis à une déshydratation dans une première colonne. Le flux gazeux distillé en tête de colonne est partiellement condensé dans un condenseur, générant un reflux liquide renvoyé à la colonne de déshydratation qui participe à l'absorption / condensation de l'acide acrylique, l'effluent gazeux non condensé étant renvoyé au moins en partie vers la réaction et le reste étant éliminé.

Le flux de pied de la colonne de déshydratation est partiellement envoyé sur une seconde colonne dite colonne de finition. L'autre partie de ce flux liquide est renvoyée en passant par un échangeur de chaleur dans la partie basse de la colonne de déshydratation, formant ainsi une boucle de recirculation. Lors de l'étape de purification/finition, on élimine en pied un flux riche en composés lourds, et en tête on récupère un distillat comprenant de l'eau et des sous-produits légers qui est condensé puis recyclé en pied de la première colonne de déshydratation. Le flux liquide provenant du pied de la colonne de déshydratation et envoyé vers la colonne de finition, contenant l'acide (méth)acrylique et les impuretés légères et lourdes issues de l'étape d'absorption - condensation, ainsi que le flux liquide provenant de la tête de colonne de finition recyclé en pied de colonne de déshydratation, contenant un flux d'acide (méth)acrylique enrichi en composés légers, forment une "boucle de recyclage" entre les 2 colonnes.

On récupère un flux d'acide acrylique purifié sous forme de liquide ou de vapeur, par soutirage latéral de la colonne de finition. L'acide acrylique obtenu est généralement de pureté supérieure à 98,5% massique et contient moins de 0,5% massique d'eau et moins de 0,4% massique d'acide acétique. Parmi les autres impuretés encore présentes, on trouve en particulier des composés lourds tels que des aldéhydes et de la protoanémonine.

L'acide acrylique purifié peut être utilisé comme acide acrylique de grade technique sans autre purification, par exemple pour produire des esters, ou peut être soumis à un traitement complémentaire par cristallisation fractionnée pour éliminer les impuretés résiduelles et conduire à une qualité d'acide acrylique de grade polymère (appelé aussi glacial).

La colonne de finition utilisable dans le procédé décrit dans le document EP 2 066 613 peut être de toute configuration, par exemple colonne à garnissage, colonne à plateaux, colonne à paroi séparatrice ; le garnissage peut être de tout type, vrac ou structuré, et le nombre de plateaux théoriques n'est pas limité.

Les conditions de fonctionnement en température et en pression pour la colonne de finition ne sont pas critiques dans ce procédé, et peuvent être déterminées conformément aux méthodes de distillation connues de l'état de l'art. Cependant, de préférence, la colonne de purification est opérée à une pression inférieure à la pression atmosphérique, permettant de fonctionner à des températures relativement faibles, évitant ainsi la polymérisation des produits insaturés présents, et minimisant la formation de sous-produits lourds.

Malgré les avantages que procure le procédé de purification décrit dans le document EP 2 066 613, il subsiste encore des inconvénients.

Pour la production de polymères d'acide (méth)acrylique ou d'esters d'acide (méth)acrylique, il est nécessaire de produire une qualité d'acide (méth)acrylique technique suffisamment débarrassée de certaines impuretés gênantes. Pour exemple, les impuretés lourdes comme le furfural, le benzaldéhyde et la protoanémonine sont gênantes parce qu'elles réagissent dans le processus de polymérisation. D'autres impuretés légères au-delà d'un certain niveau, comme l'acide acétique, peuvent générer des composés organiques volatils qui subsistent à l'intérieur du polymère d'acide (méth)acrylique et le rendent impropre à l'utilisation.

Enfin lors de la production d'esters par réaction entre l'acide (méth)acrylique de grade technique et un alcool, les impuretés ayant une fonction carboxylique, comme l'acide acétique, l'acide crotonique, ou l'acide ou l'anhydride maléique forment des impuretés difficiles à éliminer et pouvant détériorer les rendements de réaction d'estérification en consommant partiellement l'alcool mis en jeu lors de la réaction.

Afin d'éviter ces problèmes, il est nécessaire de mettre en oeuvre une colonne de finition présentant un nombre important d'étages de rectification.

L'acide acrylique est un produit très sensible à la polymérisation radicalaire, qui conduit à la formation de polymères insolubles encrassant les équipements et nécessitant des arrêts d'installation coûteux pour nettoyage.

L'addition d'inhibiteurs de polymérisation réduit cette réaction parasite, mais cette solution n'est pas suffisante pour une production continue pendant de longues périodes, en particulier lorsque la température à l'intérieur de la colonne ou de ses équipements est trop élevée. De plus, la distribution de l'inhibiteur sur les plateaux ou les garnissages de la colonne est généralement rendue difficile en raison des points morts générés par la structure, difficiles à atteindre par le reflux liquide contenant les inhibiteurs de polymérisation.

Ainsi, les opérations de distillation pour la purification de flux riches en acide acrylique sont réalisées sous pression réduite, de façon à limiter la température, et les colonnes de distillation sont généralement équipées de garnissages simples, pour permettre la distribution efficace du liquide contenant les inhibiteurs et éviter l'accumulation de germes précurseurs de polymères. Des colonnes à plateaux perforés sont par exemple utilisées.

En général, les internes de colonnes permettant de réduire les phénomènes d'initiation de la polymérisation génèrent davantage de pertes de charge par plateau théorique installé, que les colonnes plus performantes mais plus propices à la formation et à l'accumulation de polymères.

L'augmentation du nombre de plateaux de rectification pour atteindre une qualité d'acide acrylique technique améliorée conduit alors à une augmentation de la perte de charge globale de la colonne, qui se traduit par une augmentation de la température dans la colonne et une aggravation de la sensibilité à la polymérisation.

L'acide acrylique a par ailleurs la particularité de former facilement des dérivés d'addition de Michael, comme l'acide 3-acryloxypropionique, encore appelé dimère d'acide acrylique. Ces composés sont des produits lourds qui réduisent le rendement de récupération en consommant l'acide acrylique monomère.

Comme la polymérisation radicalaire, cette réaction covalente de formation de dérivés de Michael est fortement favorisée par la température. Par conséquent, la mise en place de colonnes à nombre élevé de plateaux de rectification pour satisfaire aux exigences de qualité de l'acide acrylique conduit à des inconvénients en termes de perte de produit, qui ne peuvent être compensés en partie que par un traitement additionnel de craquage à haute température des dérivés de Michael pour régénérer le monomère acide acrylique.

Ainsi, il existe un intérêt important de réaliser la séparation en diminuant le nombre de plateaux théoriques de rectification, à la fois pour réduire le risque de polymérisation et la formation de produits dérivés d'addition de Michael, ces deux effets indésirables étant favorisés par une température plus élevée.

De plus, l'élimination des impuretés légères (principalement eau et acide acétique) dans un procédé sans solvant tel que décrit dans le document EP 2 066 613 nécessite une boucle de recyclage entre les 2 colonnes dont le débit est important et inversement proportionnel à l'efficacité de la colonne de finition. Une augmentation du débit de recyclage pour compenser une efficacité insuffisante conduit ainsi à une consommation énergétique supplémentaire.

Les inventeurs ont découvert que l'utilisation d'une colonne équipée d'une paroi séparatrice et sa mise en oeuvre dans des conditions particulières, comme colonne de finition dans le procédé du document EP 2 066 613, permet de surmonter les inconvénients précités en conduisant à un gain énergétique significatif lors de la purification de l'acide acrylique tout en produisant une meilleure qualité d'acide acrylique technique.

Lorsqu'une colonne de distillation est équipée d'une paroi séparatrice, la paroi étant jointive avec le dôme supérieur de la colonne en partie haute, et non jointive avec le fond de la colonne en partie basse, la colonne comporte deux sections dont l'espace inférieur communique avec l'espace de fond de colonne et dont l'espace de tête est séparé en deux zones hermétiques.

Lorsque la colonne de finition répond à cette configuration, les inventeurs ont découvert que les composés légers et l'eau contenus dans le flux d'alimentation de la colonne de finition sont éliminés plus efficacement de la section d'alimentation, et peuvent être recyclés en pied de la colonne de déshydratation dans une boucle de recyclage de débit plus faible, tout en permettant d'extraire en tête de l'autre section l'acide acrylique avec une pureté améliorée ; et le flux de produits lourds se formant dans l'espace inférieur de la colonne est éliminé en pied de la colonne de finition.

Les inventeurs ont également découvert que dans cette configuration, lorsqu'un agent de traitement chimique des aldéhydes résiduels est introduit en amont ou dans la colonne à paroi séparatrice, il est possible de récupérer en tête de cette colonne un acide acrylique de grade polymère, cet acide acrylique de grade polymère étant de qualité supérieure, notamment en ce qui concerne la teneur résiduelle en eau, acide acétique et protoanémonine.

Les inventeurs ont également découvert que dans certaines conditions de mise en oeuvre de la colonne à paroi séparatrice, un acide acrylique de grade polymère, répondant à des spécifications concernant la teneur résiduelle en aldéhydes tels que furfural ou benzaldéhyde, et en protoanémonine peut être soutiré directement en tête de la colonne.

Il a déjà été suggéré dans la demande de brevet WO 2017/060583 au nom de la Demanderesse d'utiliser une colonne à paroi séparatrice comme colonne de finition avec la présence d'un agent de traitement chimique des aldéhydes résiduels, dans un procédé de récupération d'acide acrylique de grade polymère.

Dans ce procédé, la paroi séparatrice de la colonne de finition n'est pas jointive avec la partie haute de la colonne. La colonne de finition est alimentée en tête d'un côté de la paroi par le flux de pied de colonne de déshydratation, et un flux d'acide acrylique de grade polymère est obtenu en soutirage latéral dans la section située de l'autre côté de la paroi séparatrice. Du côté de la paroi séparatrice alimenté en flux d'acide acrylique à purifier, c'est-à-dire dans la section d'alimentation, est réalisée l'élimination des composés légers (principalement acide acétique et eau), et le flux résiduel refluant vers le fond de colonne contenant l'acide acrylique enrichi en composés lourds (dont les produits de réaction avec l'agent chimique) est distillé dans la section de soutirage située de l'autre côté de la paroi séparatrice.

L'acide acrylique purifié est recueilli en soutirage latéral dans cette deuxième section. Le flux gazeux obtenu en tête de cette section de soutirage est mélangé avec le flux gazeux comportant les composés légers éliminés en tête de la section d'alimentation, puis renvoyé sous forme liquide, après condensation, dans la boucle de recirculation du fond de la colonne de déshydratation. Le flux liquide de pied de colonne de déshydratation alimentant la colonne de finition et le flux de tête de colonne de finition recyclé vers la colonne de déshydratation constituent une boucle de recyclage contenant majoritairement de l'acide acrylique.

L'acide acrylique obtenu selon ce procédé est de grade polymère, ayant une teneur en poids en acide acrylique > 99 %, de préférence > 99,5 %, et comportant une teneur en aldéhydes totaux < 10 ppm, voire < 3 ppm. Il contient en outre moins de 5 ppm de protoanémonine.

Dans le schéma de purification décrit dans la demande de brevet WO 2017/060583 , pour obtenir un flux d'acide acrylique suffisamment débarrassé de l'eau et de l'acide acétique, il est nécessaire de maintenir un débit suffisant de flux de tête enrichi en composés légers de la colonne de finition vers la colonne de déshydratation. Ce flux de tête est majoritairement constitué d'acide acrylique qu'on souhaite récupérer en totalité. Le recyclage d'un flux important de tête de colonne de finition vers la colonne de déshydratation s'accompagne d'un débit d'alimentation accru du flux de pied de colonne de déshydratation vers l'alimentation de la colonne de finition. Par conséquent, c'est le débit de l'ensemble de la boucle de recyclage qui se trouve impacté, et il est nécessaire de disposer de l'énergie suffisante pour assurer la vaporisation de la boucle de recyclage. Dans ces conditions seulement, il est possible d'éviter de « polluer » avec des composés légers l'acide acrylique du soutirage latéral et garantir une qualité constante d'acide acrylique de grade polymère.

Par ailleurs, les agents chimiques utilisés pour l'élimination des aldéhydes ne sont pas assez réactifs pour l'élimination de l'impureté protoanémonine, qui est un composé lourd de type lactone. L'élimination complète de la protoanémonine jusqu'à de très faibles teneurs, afin d'obtenir une qualité d'acide (méth)acrylique glacial compatible avec la fabrication de polymères de hautes masses moléculaires, requiert une efficacité de colonne très grande. La présence de protoanémonine dans l'acide acrylique de grade polymère, même à une concentration aussi faible que 5 ppm, voire 3 ppm, peut être rédhibitoire pour la fabrication de polymères d'acide acrylique ou de sels d'acide acrylique de hautes masses moléculaires.

Or, cette élimination de la protoanémonine reste un problème non résolu dans ce procédé. En effet, l'obtention du produit purifié en soutirage latéral, c'est-à-dire en un point situé plus bas que la tête de la section de soutirage, réduit la hauteur de la section de séparation située en-dessous de ce soutirage. Par conséquent, pour obtenir un flux d'acide acrylique totalement exempt de protoanémonine, il est nécessaire de mettre en place une colonne de très grande efficacité. Les inconvénients générés sont le coût d'investissement, une augmentation de la formation d'acide 3-acryloxypropionique (dimère AA) et un risque de polymérisation accru, puisque l'addition de plateaux de rectification d'une colonne à distiller provoque une augmentation de la perte de charge totale de la colonne et par conséquent une augmentation de la température en fond de colonne.

Il subsiste donc un besoin d'améliorer l'élimination de l'impureté protoanémonine dans les procédés de récupération d'acide acrylique de grade polymère décrits dans l'art antérieur.

Il a maintenant été découvert qu'une colonne de finition équipée d'une paroi séparatrice mise en oeuvre dans des conditions particulières, permet de répondre à ce besoin, et conduit à un acide acrylique de grade polymère comportant moins de 2 ppm de protoanémonine, une teneur massique en eau inférieure à 0,1 % et une teneur massique en aldéhydes inférieure à 3 ppm..

### RESUME DE L'INVENTION

La présente invention a pour objet un procédé de récupération d'acide (méth)acrylique purifié, en l'absence de solvant organique, à partir d'un mélange réactionnel gazeux comprenant de l'acide (méth)acrylique obtenu par oxydation en phase gazeuse d'un précurseur de l'acide (méth)acrylique, comprenant au moins les étapes suivantes :
a) le mélange réactionnel gazeux est soumis à une déshydratation sans utiliser de solvant azéotropique dans une première colonne de distillation dite colonne de déshydratation, conduisant à un flux de tête dont une partie au moins est condensée et renvoyée à la colonne de déshydratation sous forme de reflux, et à un flux de pied dont une partie au moins est renvoyée en reflux dans la partie inférieure de la colonne de déshydratation pour former une boucle de recirculation ;
b) le flux de pied de la colonne de déshydratation est envoyé au moins en partie dans une seconde colonne de distillation dite colonne de finition, permettant de séparer un flux de pied contenant des composés lourds et un flux de tête contenant des composés légers, dont une partie au moins est renvoyée dans la colonne de déshydratation;
ledit procédé étant caractérisé en ce que :
i) la colonne de finition est équipée d'une paroi séparatrice, la paroi étant jointive avec le dôme supérieur de la colonne en partie haute et non jointive avec le fond de la colonne en partie basse, séparant ainsi la colonne en deux sections équipées d'éléments internes de distillation assurant le contact gaz-liquide, dont l'espace inférieur communique avec l'espace de fond de colonne, et dont l'espace de tête est séparé en deux zones hermétiques, l'alimentation de ladite colonne s'effectuant d'un seul côté de la paroi séparatrice, et
ii) un flux gazeux riche en composés légers et comprenant de l'eau et de l'acide acétique est extrait en tête de la section d'alimentation, puis recyclé, après condensation, au moins en partie dans la boucle de recirculation en pied de la colonne de déshydratation, et
iii) un flux d'acide (méth)acrylique purifié extrait sous forme gazeuse de la colonne de finition en tête de la section située de l'autre côté de la section d'alimentation, est soutiré après condensation, une partie du flux condensé étant renvoyée comme reflux liquide en tête de la section de soutirage.

Par souci de simplification dans la suite de l'exposé de l'invention, par « section d'alimentation », on entend la section de la colonne à paroi séparatrice qui est alimentée par le flux d'acide (méth)acrylique à purifier ; et par « section de soutirage », on entend la section de la colonne à paroi séparatrice d'où est extrait en tête le flux d'acide (méth)acrylique purifié.

Selon un premier mode de réalisation particulier, l'alimentation de la colonne de finition s'effectue en tête au niveau du plateau supérieur de la section d'alimentation, et de manière optionnelle, une partie du flux gazeux, qui est extrait en tête de la section d'alimentation, est renvoyée après condensation, dans le flux d'alimentation de la colonne de finition.

Selon un second mode de réalisation particulier, l'alimentation de la colonne de finition s'effectue en un point situé plus bas que le plateau supérieur de la section d'alimentation, et une partie du flux gazeux qui est extrait en tête de la section d'alimentation, est renvoyée après condensation comme reflux liquide en tête de la section d'alimentation.

Selon ces deux modes de réalisation, le procédé selon l'invention permet de produire un flux d'acide (méth)acrylique purifié de meilleure qualité, tout en permettant de diminuer le débit de la boucle de recyclage composée du pied de la colonne de déshydratation et de la tête de colonne de finition, et donc de réduire l'énergie associée.

Le flux l'acide (méth)acrylique purifié extrait en tête de la colonne de finition peut être utilisé directement comme acide (méth)acrylique de grade technique sans autre purification.

En particulier, l'acide (méth)acrylique de grade technique présente avantageusement les teneurs massiques en impuretés suivantes :
Eau : < 0,2%, de préférence < 0,05%, plus préférentiellement < 0,01%
Acide acétique : < 0,2%, de préférence < 0,05%, plus préférentiellement < 0,02%
Furfural : < 0,05%, de préférence < 0,02%, plus préférentiellement < 0,005%
Benzaldéhyde : < 0,05%, de préférence < 0,02%,plus préférentiellement < 0,005%
Protoanémonine : < 0,05%, de préférence < 0,02%, plus préférentiellement < 0,005%.

Cet acide acrylique technique peut être soumis à un traitement complémentaire par cristallisation fractionnée, ou par distillation éventuellement en présence d'un composé réagissant avec les aldéhydes résiduels, conduisant à une qualité d'acide (méth)acrylique de grade polymère. Du fait de la qualité technique améliorée par rapport aux procédés de l'art antérieur, la purification complémentaire pour produire un grade polymère se trouve simplifiée.

Le procédé selon l'invention peut comprendre en outre l'introduction dans la colonne de finition, d'un agent de traitement chimique visant à réduire la teneur en aldéhydes résiduels, le flux d'acide (méth)acrylique extrait en tête de la colonne de finition étant alors un flux d'acide (méth)acrylique de grade polymère.

Selon certains modes de réalisation particuliers, le procédé selon l'invention conduisant à un acide (méth)acrylique de grade polymère peut présenter également au moins une des caractéristiques avantageuses énumérées ci-dessous :
- l'agent de traitement chimique est introduit dans le flux d'alimentation de la colonne de finition ;
- l'agent de traitement chimique est introduit par l'intermédiaire d'un dispositif de mélange comprenant au moins une capacité assurant la dispersion efficace de l'agent chimique avec le flux d'alimentation ;
- l'agent de traitement chimique est introduit directement dans la colonne de finition en un point situé entre la tête et le pied de colonne, plus bas que le plateau où est effectuée l'alimentation de la colonne, de préférence, en un point situé entre environ un tiers et deux tiers de la hauteur de la section d'alimentation de la colonne de finition.
- un soutirage latéral d'un flux gazeux comprenant de l'acide (méth)acrylique de qualité intermédiaire est effectué à partir de la section de soutirage.

En alternative, en adaptant le nombre de plateaux de la colonne à paroi séparatrice, notamment un nombre de plateaux théoriques allant de 15 à 20 pour chacune des sections d'alimentation et de soutirage, il est possible de produire directement en tête de la colonne de finition un acide (méth) acrylique de grade polymère sans avoir recours à un agent de traitement chimique des aldéhydes.

Dans toutes ses variantes, l'acide (méth)acrylique de grade polymère soutiré en tête de la colonne de finition, a une teneur en poids en acide (méth)acrylique > 99,5 %, et comporte moins de 2 ppm de protoanémonine, de préférence moins de 1 ppm de protoanémonine, et moins de 3 ppm de préférence moins de 1 ppm d'aldéhydes totaux (furfural et benzaldéhyde). La teneur massique en eau est généralement inférieure à 0,05%, de préférence inférieure à 0,01%, la teneur massique en acide acétique est généralement inférieure à 0,05%, de préférence inférieure à 0,02%.

Un autre objet de l'invention est un procédé de production d'acide (méth)acrylique purifié comprenant au moins les étapes suivantes :
A) on soumet à une oxydation en phase gazeuse au moins un précurseur d'acide (méth)acrylique pour former un mélange réactionnel gazeux comprenant de l'acide (méth)acrylique ;
B) on refroidit le mélange réactionnel gazeux ;
C) on soumet le mélange réactionnel gazeux refroidi au procédé de récupération de l'acide (méth)acrylique tel que défini précédemment.

Le procédé selon l'invention peut comprendre en outre d'autres étapes préliminaires, intermédiaires ou subséquentes pour autant qu'elles n'affectent pas négativement l'obtention de l'acide (méth)acrylique purifié.

Selon un mode réalisation de l'invention, le précurseur de l'acide (méth)acrylique est l'acroléine.

Selon un mode réalisation de l'invention, l'acroléine est obtenue par oxydation de propylène ou par oxydéshydrogénation de propane.

Selon un mode réalisation de l'invention, le précurseur de l'acide (méth)acrylique est la méthacroléine.

Selon un mode réalisation de l'invention, la méthacroléine est obtenue par oxydation d'isobutylène et/ou de tert-butanol.

Selon un mode réalisation de l'invention, la méthacroléine est obtenue à partir d'oxydéshydrogénation de butane et/ou isobutane.

Selon un mode réalisation de l'invention, le mélange réactionnel gazeux comprenant de l'acide (méth)acrylique obtenu par oxydation en phase gazeuse d'un précurseur de l'acide (méth)acrylique comprend du carbone d'origine renouvelable.

Selon un mode réalisation de l'invention, le précurseur de l'acide (méth)acrylique est dérivé du glycérol, de l'acide 3-hydroxypropionique ou de l'acide 2-hydroxypropionique (acide lactique).

Selon un mode réalisation préféré de l'invention, le mélange réactionnel gazeux comprend de l'acide acrylique dérivé de propylène obtenu selon un procédé d'oxydation en deux étapes.

Dans un mode de réalisation utilisant un agent de traitement des aldéhydes résiduels, le procédé selon l'invention produit un flux d'acide (méth)acrylique de grade polymère, correspondant à une qualité supérieure pour produire des polymères de haute masse moléculaire, utilisables par exemple comme superabsorbants.

Dans un mode de réalisation préféré, le procédé selon l'invention produit un flux d'acide (méth)acrylique de grade polymère sans avoir recours à un agent de traitement des aldéhydes résiduels.

Le procédé selon l'invention ne nécessite pas l'utilisation d'un solvant organique externe pour éliminer l'eau contenue dans le mélange réactionnel gazeux comprenant l'acide (méth)acrylique. Il ne nécessite pas de traitement complémentaire par cristallisation, coûteux en énergie.

Le procédé selon l'invention ne met en oeuvre qu'une colonne de déshydratation et une colonne de finition pouvant inclure éventuellement une étape de traitement des aldéhydes à l'aide d'un agent chimique, réalisée à l'intérieur d'une colonne de finition comportant une paroi séparatrice dans une configuration particulière. La séparation physique via la paroi séparatrice, entre le flux d'alimentation et l'acide purifié conduit à une augmentation du nombre d'étages théoriques, pour une hauteur de colonne de finition et un assemblage d'éléments internes de distillation fixés, d'une part pour séparer efficacement les composés légers et obtenir un gain énergétique pour le recyclage, d'autre part pour séparer efficacement les composés lourds et obtenir une meilleure qualité d'acide (méth)acrylique purifié.

Il en résulte aussi une diminution de la quantité d'agent chimique nécessaire pour éliminer les aldéhydes résiduels qui sont séparés de façon plus efficace dans la colonne de finition, et même une élimination efficace des aldéhydes résiduels sans nécessiter l'introduction d'un agent chimique.

Selon l'invention, il est possible de garantir une qualité constante d'acide acrylique de grade polymère avec un coût de purification optimisé.

D'autres caractéristiques et avantages de l'invention ressortiront mieux à la lecture de la description détaillée qui suit, en référence aux figures 1 à 4 annexées qui représentent :
- Figure 1 : Installation adaptée à la mise en oeuvre du procédé de l'art antérieur décrit dans le brevet EP 2 066 613.
- Figure 2 : Installation adaptée à la mise en oeuvre du procédé de récupération d'acide (méth)acrylique purifié selon un premier mode de réalisation de l'invention, avec l'ajout éventuel d'un agent de traitement chimique.
- Figure 3 : Installation adaptée à la mise en oeuvre du procédé de récupération d'acide (méth)acrylique purifié selon un second mode de réalisation de l'invention, avec l'ajout éventuel d'un agent de traitement chimique.
- Figure 4 : Installation selon l'art antérieur adaptée à la mise en oeuvre d'un procédé de récupération d'acide (méth)acrylique de grade polymère utilisant une colonne de distillation à paroi séparatrice comme colonne de finition.

### DESCRIPTION DETAILLEE DE L'INVENTION

Dans la présente invention, le terme « (méth)acrylique » signifie « acrylique » ou « méthacrylique ». Par souci de simplification, la suite de l'exposé fera référence à la production d'acide acrylique, mais s'applique également par analogie à la production d'acide méthacrylique.

Le terme « solvant organique externe » désigne tout composé organique dans lequel l'acide (méth)acrylique est soluble et dont l'origine est extérieure au procédé, utilisé comme solvant d'absorption, d'extraction ou de distillation azéotropique

Le terme « solvant azéotropique » désigne tout solvant organique présentant la propriété de former un mélange azéotropique avec l'eau.

Le terme « non condensable » ou « incondensable » désigne les composés dont le point d'ébullition est inférieur à la température de 20°C sous la pression atmosphérique.

Le terme « léger », qualifiant les composés sous-produits, désigne les composés dont le point d'ébullition est inférieur à celui de l'acide (méth)acrylique sous la pression de travail considérée, et par analogie, le terme « lourd » désigne les composés dont le point d'ébullition est supérieur à celui de l'acide (méth)acrylique.

Le terme « agent de traitement chimique des aldéhydes » signifie un composé chimique qui forme avec les aldéhydes des produits de réaction plus lourds qui sont plus facilement séparables de l'acide (méth)acrylique par distillation, permettant ainsi de réduire jusqu'à un niveau très faible le taux d'aldéhydes présents dans le milieu à traiter.

Par « traitement chimique » on entend le traitement réalisé à l'aide de l'agent de traitement chimique des aldéhydes.

Ce type de traitement et les composés utilisables sont bien connus dans l'état de l'art, sans que les réactions ou complexations mises en oeuvre soient complètement identifiées. Le mode d'action a essentiellement pour but de former des produits de réaction plus lourds que les aldéhydes à traiter.

Ce terme d'agent de traitement chimique des aldéhydes exclut les inhibiteurs de polymérisation qui, même s'ils peuvent avoir un effet mineur sur les aldéhydes, sont généralement introduits dans le seul but de stabiliser les flux contenant des dérivés (méth)acryliques vis-à-vis d'une polymérisation, ces inhibiteurs de polymérisation pouvant être introduits à différents niveaux et/ou dans différents flux de l'installation.

Le terme « grade polymère » et le terme « glacial » ont la même signification et indiquent que l'acide (méth)acrylique répond à des critères de haute qualité permettant son utilisation dans la fabrication de polymères (méth)acryliques de haute masse moléculaire.

Par « boucle de recyclage » entre les 2 colonnes, on entend la boucle formée par le flux liquide provenant du pied de la colonne de déshydratation et envoyé vers la colonne de finition, et par le flux liquide provenant de la tête de colonne de finition recyclé en pied de colonne de déshydratation.

L'invention vise à produire de l'acide acrylique de haute pureté avec un coût de purification optimisé, et elle est basée sur la mise en oeuvre dans des conditions particulières d'une colonne équipée d'une paroi séparatrice comme colonne de finition dans un procédé de purification de l'art antérieur impliquant un nombre réduit de colonnes de distillation et ne nécessitant pas de solvant organique externe.

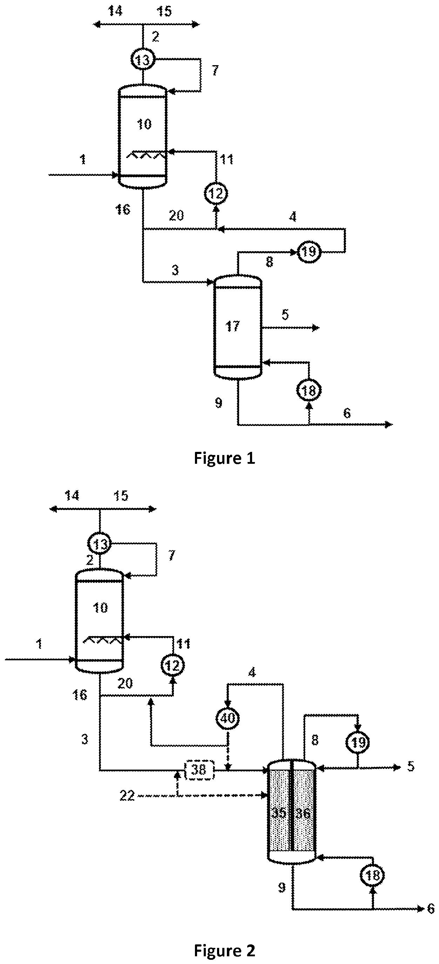

Selon ce procédé de l'art antérieur représenté à la Figure 1, un mélange réactionnel gazeux 1 comprenant de l'acide acrylique obtenu par oxydation en phase gazeuse d'un précurseur de l'acide acrylique alimente une première colonne de distillation 10. Le mélange réactionnel gazeux comportant un rapport massique eau/acide acrylique généralement compris entre 0,3 et 2, de préférence entre 0,3 et 1,2, peut être préalablement refroidi avant d'être soumis à une déshydratation dans la colonne de déshydratation 10.

Le mélange réactionnel comprend en plus de l'eau et l'acide acrylique, des produits légers incondensables tels que l'azote, l'oxygène, le monoxyde et le dioxyde de carbone, ainsi que différents sous-produits légers ou lourds de différente nature chimique, pouvant être des aldéhydes légers comme l'acroléine, le formaldéhyde, l'acétaldéhyde ou le glyoxal, des aldéhydes lourds tels que le furfuraldéhyde ou le benzaldéhyde, des acides légers tels que l'acide formique, l'acide acétique ou l'acide propionique, des acides lourds tels que l'acide maléique, l'acide benzoïque ou l'acide 2-butènoïque, et de la protoanémonine composé lourd de type lactone.

La colonne de déshydratation conduit à un flux de tête 2 dont au moins une partie est condensée dans un condenseur 13 et renvoyée à la colonne de déshydratation sous forme de reflux 7 pour absorber l'acide acrylique, l'autre partie (flux 14 et 15) comprenant les composés légers incondensables étant généralement envoyée partiellement ou totalement à un dispositif d'épuration ou recyclée en partie vers d'autres étapes du procédé de production d'acide acrylique, de préférence dans une étape située en amont du réacteur de production du mélange réactionnel 1.

La totalité du flux de tête de la colonne de déshydratation peut être envoyée dans le condenseur de tête 13.

L'étape de déshydratation a pour but d'éliminer dans un flux de tête l'essentiel de l'eau présente dans le mélange réactionnel, mais aussi les composés légers incondensables et les composés légers condensables. Elle génère un flux de tête 2 comprenant l'essentiel de l'eau et des composés légers, avec de l'acide acrylique et des composés lourds en quantité très faible, et un flux de pied 16 appauvri en composés légers comprenant la quasi-totalité de l'acide acrylique avec des sous-produits lourds, et une teneur massique en eau généralement inférieure à 10%, de préférence inférieure à 7%.

Une composition massique typique du flux de pied 16 de la colonne de déshydratation comprend essentiellement de l'acide acrylique (84-90%), de l'acide acétique (2-10%), de l'eau (2-10%), et des sous-produits lourds.

La colonne de déshydratation comprend généralement de 5 à 50 plateaux théoriques, de préférence de 20 à 30 plateaux théoriques.

Avantageusement, la colonne de déshydratation fonctionne à la pression atmosphérique ou légèrement supérieure, jusqu'à une pression absolue de 1,5 10⁵ Pa.

Avantageusement, la température dans la partie supérieure de la colonne de déshydratation est d'au moins 40°C, de préférence est comprise entre 40°C et 80°C. La température du flux de pied de la colonne de déshydratation ne dépasse pas de préférence 120°C.

Le flux de pied 16 de la colonne de déshydratation est envoyé au moins en partie (flux 3), en tête d'une seconde colonne de distillation 17, dite colonne de purification ou colonne de finition, dans laquelle sont séparés un flux de tête 8 et un flux de pied 9.

Une partie 20 du flux liquide 16 de pied de la colonne de déshydratation est envoyée dans un échangeur de chaleur 12 qui peut être un réchauffeur ou un refroidisseur et réinjectée dans la colonne de déshydratation, de façon à constituer une boucle de recirculation en pied. De préférence, la partie 11 de la boucle de pied est réinjectée entre l'alimentation du mélange gazeux réactionnel et la tête de colonne de déshydratation.

Le reste (flux 3) du flux liquide 16 est envoyé en alimentation de la colonne de finition 17.

La colonne de finition 17 est généralement une colonne de distillation classique comprenant de 5 à 30 plateaux théoriques, de préférence de 8 à 20 plateaux théoriques. Cette colonne de distillation est associée en pied à au moins un rebouilleur 18 et en tête à un condenseur 19.

La température et la pression dans la colonne 17 ne sont pas critiques, et peuvent être déterminées conformément aux méthodes de distillation connues de l'état de l'art. Cependant, de préférence, la colonne de finition 17 fonctionne à une pression inférieure à la pression atmosphérique, permettant de fonctionner à des températures relativement faibles, évitant ainsi la polymérisation des produits insaturés présents, et minimisant la formation de sous-produits lourds.

Avantageusement, la colonne de finition fonctionne sous une pression absolue allant de 5 kPa à environ 60 kPa, la température du flux de tête étant avantageusement comprise entre 40°C et environ 90°C, et la température du flux de pied étant comprise entre 60°C et 120°C.

Le flux gazeux de tête 8 de la colonne de finition est envoyé dans le condenseur 19, et le flux liquide sortant 4 est renvoyé vers la colonne de déshydratation, mélangé au flux de la boucle de pied de la colonne de déshydratation. Le flux de tête 8 comprend essentiellement de l'eau et les sous-produits légers condensables.

Le flux 9 séparé en pied de la colonne de finition comprend l'essentiel des sous-produits lourds, notamment des produits d'addition de Michael tels que l'acide 3-acryloxypropionique, de l'anhydride/acide maléique, de l'acide benzoïque, ainsi que des inhibiteurs de polymérisation. Ce flux 9 peut être en partie recyclé dans le bas de la colonne de finition, ou utilisé comme matière première pour préparer des esters acryliques (flux 6).

Un flux 5 comprenant de l'acide acrylique purifié sous forme de liquide ou de vapeur, de préférence gazeux, est extrait de la colonne de finition par soutirage latéral. Ce flux 5 correspond à de l'acide acrylique de grade technique.

La Figure 2 représente une installation adaptée à la mise en oeuvre du procédé de récupération d'acide (méth)acrylique purifié selon un premier mode de réalisation de l'invention.

Cette installation se différencie de l'installation de l'art antérieur en ce qu'elle comprend comme colonne de finition 17, une colonne équipée d'une paroi séparatrice, la paroi étant jointive avec le dôme supérieur de la colonne en partie haute et non jointive avec le fond de la colonne en partie basse. La paroi sépare ainsi la colonne en deux sections 35 et 36 équipées d'éléments de rectification. L'espace inférieur de la colonne situé sous la paroi séparatrice peut être vide ou de préférence équipé de quelques éléments de rectification (non représentés sur la figure) communiquant avec l'espace de fond de colonne. L'espace de tête est séparé par cette paroi en deux zones vides non jointives.

Le nombre de plateaux théoriques nécessaire à la séparation des composés légers, dans la section d'alimentation 35 est généralement compris entre 5 et 20, de préférence entre 15 et 20, et l'espace inférieur commun aux 2 sections peut comprendre entre 1 et 5 plateaux théoriques. Dans la section de soutirage 36, le nombre de plateaux théoriques est généralement compris entre 2 et 20, de préférence entre 15 et 20 plateaux théoriques.

Selon un mode de réalisation préféré, le nombre de plateaux théoriques dans chacune des sections d'alimentation et de soutirage est compris entre 15 et 20, et l'espace inférieur commun situé sous la paroi séparatrice est équipé d'éléments de rectification représentant entre 1 et 5 plateaux théoriques. Selon ce mode de réalisation, il n'est pas nécessaire d'introduire un agent de traitement chimique des aldéhydes pour récupérer en tête de colonne un acide acrylique de grade polymère.

Les sections de la colonne peuvent être équipées de tous types d'internes, par exemple : garnissages vrac ou ordonnés, plateaux perforés sans déversoirs ou avec déversoirs comme les plateaux à cloches, plateaux à clapets fixes ou mobiles, etc.

L'alimentation de la colonne de finition s'effectue d'un seul côté de la paroi séparatrice, dans la section d'alimentation 35.

Le flux d'acide acrylique brut 3 obtenu en pied de la colonne de déshydratation 10, est envoyé dans la section 35 en tête de la colonne de finition.

Selon ce premier mode de réalisation, l'alimentation de la colonne de finition s'effectue en tête au niveau du plateau supérieur de la section d'alimentation.

Un flux gazeux 4 riche en composés légers et comprenant de l'eau est extrait en tête de la section 35, condensé dans un échangeur 40 et recyclé au moins en partie dans la boucle de recirculation en pied de la colonne de déshydratation, dans le flux 20.

Une partie du flux 4 qui est extrait en tête de la section d'alimentation, peut être avantageusement renvoyée après condensation dans le flux d'alimentation de la colonne de finition.

Le flux obtenu en pied de section 35 contenant l'acide acrylique, débarrassé des composés légers et de l'eau, est purifié dans la section 36 en vue d'éliminer en pied un flux 9 riche en composés lourds. Ce flux 9 est en partie recyclé dans le bas de la colonne de finition, à travers un rebouilleur 18, et le complément 6 est soutiré. Ce flux soutiré (flux 6) contient de l'acide acrylique résiduel et des composés lourds du type dérivés d'addition de Michael (acide 3-acryloxypropionique). De manière avantageuse, il pourra être envoyé dans une section de récupération dans le but de récupérer l'acide acrylique, par exemple par distillation dans un évaporateur, ou par craquage thermique dans un réacteur à haute température, de façon à régénérer le monomère à partir des dérivés de Michael, ou en combinant ces deux types d'équipements._De manière alternative, le flux 9 peut également être utilisé comme matière première riche en acide acrylique et en produit lourds dérivés de réactions de Michael pour la synthèse d'esters, notamment dans des conditions propices au craquage de ces lourds et à la régénération consécutive du monomère.

De façon générale , la colonne de finition à paroi séparatrice va opérer sous vide dans un domaine de pression allant de 5 kPa à 60 kPa, (50 mbar à 600 mbar) de préférence entre 5 et 20 kPa, avec une température en tête de colonne comprise entre 50°C et 80°C , et une température en pied de colonne comprise entre 85°C et 120°C. Le ratio du débit massique entre le soutirage en tête de colonne (5) et le débit de pied (6) est au moins de 75/25, préférentiellement au moins de 95/5. Par ailleurs, le ratio massique du débit de retour du flux de tête de la colonne de finition sur la colonne de déshydratation (flux 4) sur la somme des flux soutirés en tête (5) et en pied de colonne (6) est généralement compris entre 1 et 4.

En tête de la section 36, un flux gazeux 8 d'acide acrylique purifié est extrait de la colonne de finition. Une partie de ce flux, après condensation, est additionnée d'inhibiteurs de polymérisation, puis renvoyée comme reflux liquide en tête de la section de soutirage. Les inhibiteurs de polymérisation utilisés sont de nature et en concentration adaptées pour l'usage de l'acide acrylique technique ainsi récupéré.

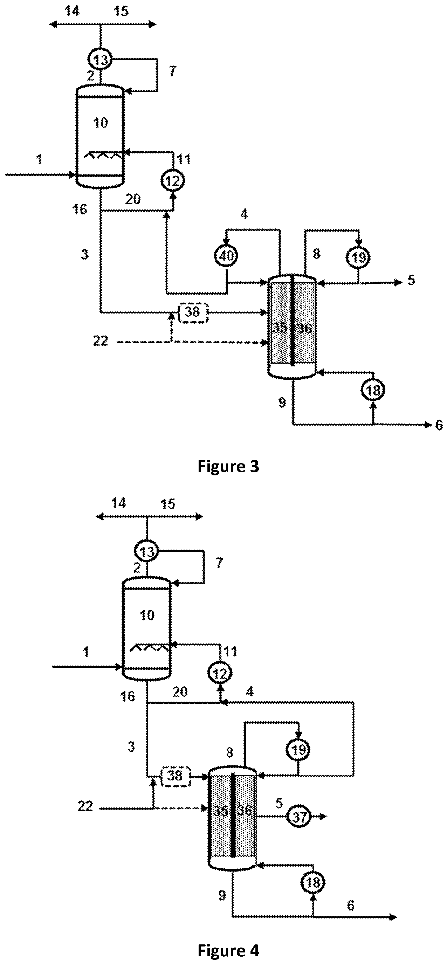

La Figure 3 représente une installation adaptée à la mise en oeuvre du procédé de récupération d'acide (méth)acrylique purifié selon un second mode de réalisation de l'invention.

Selon ce second mode de réalisation, l'alimentation de la colonne de finition est effectuée en un point situé plus bas que le plateau supérieur de la section d'alimentation, de préférence en un point situé entre le quart et les trois quarts de la hauteur de la section d'alimentation et une partie du flux gazeux 4 qui est extrait en tête de la section d'alimentation, est renvoyée après condensation comme reflux liquide en tête de la section d'alimentation.

De la même façon que le premier mode de réalisation de l'invention, le flux obtenu en pied de section 35 contenant l'acide acrylique, débarrassé des composés légers et de l'eau, est purifié dans la section 36 en vue d'éliminer en pied un flux 9 riche en composés lourds. Ce flux 9 est en partie recyclé dans le bas de la colonne de finition, à travers un rebouilleur 18, et le complément est soutiré (flux 6), pour être envoyé de manière avantageuse dans une section de récupération du monomère résiduel présent sous forme libre ou combinée (acide 3-acryloxypropionique), ou dans une unité de production d'esters acryliques, comme il est décrit dans le premier mode de réalisation de l'invention.

En tête de la section 36, un flux gazeux 8 d'acide acrylique purifié est extrait de la colonne de finition. Une partie de ce flux, après condensation, est additionnée d'inhibiteurs de polymérisation, puis renvoyée comme reflux liquide en tête de la section de soutirage. Les d'inhibiteurs de polymérisation utilisés sont de nature et en concentration adaptées pour l'usage de l'acide acrylique technique ainsi récupéré.

Selon ces deux modes de réalisation de l'invention représentés sur les Figures 2 et 3, un dispositif de récupération de solides (non représenté sur les figures), par exemple un filtre, peut être placé en ligne ou en dérivation sur la boucle de recirculation en pied de la colonne de finition, afin d'éviter les encrassements potentiels de la colonne de finition.

Comparativement à la configuration utilisant une colonne de finition classique représentée à la Figure 1, l'invention selon les deux modes de réalisation permet de mettre en oeuvre, pour un même nombre de plateaux installés, une hauteur de colonne plus faible fonctionnant à une pression et une température plus faibles ; il s'ensuit que l'acide acrylique peut être produit de manière fiable et continue, sans interruption causée par le dépôt de solides polymériques sur les équipements. De plus, l'ajout d'inhibiteur de polymérisation peut être réalisé en quantité moindre pour éviter la formation de polymères d'acide acrylique dans la colonne. Par ailleurs, pour une même hauteur de colonne, l'invention conduit à une meilleure qualité d'acide acrylique technique et une diminution du débit de la boucle de recyclage en tête de colonne de finition.

Lorsqu'un agent de traitement chimique visant à réduire la teneur en aldéhydes résiduels est introduit en outre dans la colonne de finition à paroi séparatrice, représenté par le flux 22, sur les Figures 2 et 3, l'acide acrylique purifié extrait en tête de la section de soutirage 36 correspond directement à une qualité d'acide acrylique de grade polymère.

Les agents de traitement chimique des aldéhydes utilisables dans l'invention peuvent être ceux décrits dans l'art antérieur pour des procédés combinant une purification par distillation et traitement chimique des aldéhydes contenus dans un acide acrylique technique. Il peut s'agir d'agents chimiques mis en oeuvre seuls ou en mélange en toutes proportions.

On peut citer notamment :
- des amines, telles que par exemple, sans limitation, la monoéthanolamine, l'éthylène diamine la glycine, la diéthylènetriamine, la dipropylènetriamine, l'ortho-, la para-, et la meta-phenylenediamine ;
- des composés de la famille de l'aniline, comme par exemple, sans limitation, l'aniline, l'ortho-, la para-, et la meta-méthylaniline ;
- des composés de la famille des hydrazines, tel que, sans limitation, l'hydrazine et ses sels, l'hydrate d'hydrazine, le sulfate d'hydrazine, les carboxylates d'hydrazine, le chlorhydrate d'hydrazine, la phénylhydrazine, la 4-nitrophénylhydrazine, et la 2,4-dinitrophénylhydrazine, ou encore l'aminoguanidine et ses sels, comme l'hydrogénocarbonate d'aminoguanidine ;
- Des composés de la famille des hydrazides, comme par exemple, sans limitation, les hydrazides d'acides carboxyliques et leurs sels, tels que les hydrazides d'acides formique, acétique, propionique, butanoïque, pentanoïque, maléique et les dihydrazides d'acides adipique et succinique, l'urée ou des dérivés de l'urée et de l'hydrazine, comme le semicarbazide ou le carbohydrazide et leurs sels ;
seuls ou leurs mélanges en toutes proportions.

De préférence, on utilise dans l'invention, l'hydrazine ou un dérivé d'hydrazine, ou un sel d'aminoguanidine, tel que l'hydrogénocarbonate d'aminoguanidine pour réduire la teneur en aldéhydes résiduels.

Les agents chimiques sont introduits tels quels dans le flux à traiter, ou en solution dans un solvant, par exemple en solution dans de l'acide acrylique.

L'agent chimique est introduit en quantité minimale pour obtenir une qualité d'acide acrylique glacial suffisamment débarrassée des impuretés aldéhydiques (en particulier acroléine, furfuraldéhyde et benzaldéhyde) pour répondre aux besoins des clients. En général, l'agent chimique est ajouté dans un rapport molaire de 0,5 à 10, de préférence de 1 à 5, par rapport à la totalité des aldéhydes présents dans le milieu à traiter.

L'agent chimique peut être introduit dans le flux qui alimente la section d'alimentation 35, par l'intermédiaire d'un dispositif de mélange 38.

Selon ce mode de réalisation de l'invention (représenté sur la Figure 2 et la Figure 3), le mélange de l'agent de traitement chimique des aldéhydes avec le flux 3 est réalisé en amont de la colonne de finition, dans un dispositif de mélange 38 permettant la dispersion la plus efficace de l'agent de traitement chimique dans le flux. Ce dispositif peut en particulier contenir en série une ou plusieurs capacités et un ou plusieurs équipements de mélange ou d'échange de chaleur, de façon à réaliser le traitement à une température et pendant un temps de séjour optimums. De manière non exhaustive, les équipements de mélange peuvent inclure des outils généralement utilisés par l'homme de l'art pour mélanger des liquides, comme des récipients agités ou recirculés ou des mélangeurs statiques, mais aussi tout type d'équipements permettant une dispersion rapide de l'agent chimique de traitement dans le flux à traiter, comme des mélangeurs à jet axial, à jet rotatif, éjecteurs à jet liquide, hydroéjecteurs, pompes, filtres, etc.

En alternative, l'agent chimique peut être introduit directement dans la colonne de finition en un point situé entre la tête et le pied de colonne, plus bas que le plateau où est effectuée l'alimentation de la colonne, de préférence, en point situé entre environ un tiers et deux tiers de la section d'alimentation de la colonne de finition.

Il est en effet nécessaire, pour des facilités d'opération, de limiter le risque de formation de solides à une zone très proche du fond de la colonne, et éventuellement de placer un système permettant de recueillir et d'éliminer facilement le solide formé.

De plus, les aldéhydes légers étant au moins partiellement éliminés en tête par distillation en pénétrant dans la colonne de finition fonctionnant sous pression réduite, la concentration en aldéhydes totaux est plus faible dans la partie située entre environ un tiers et deux tiers de la section d'alimentation de la colonne de finition, la quantité d'agents chimiques à introduire est donc plus faible, voire nulle.

Le flux 9 séparé en bas de la colonne de finition est alors un flux riche en composés lourds initialement contenus dans le flux d'alimentation 3, ou formés lors des réactions d'élimination des impuretés avec l'agent chimique.

Selon l'invention, l'augmentation du nombre d'étages de séparation entre le flux d'alimentation et celui permettant de récupérer l'acide acrylique de grade polymère, conduit à une colonne de très grande efficacité permettant d'éliminer complètement l'impureté protoanémonine et quasi complètement les impuretés aldéhydes sans même nécessiter de traitement à l'aide d'un agent chimique.

A titre de comparaison, sur la Figure 4 est représentée une colonne de distillation à paroi séparatrice comme colonne de finition, dans un procédé de récupération d'acide (méth)acrylique de grade polymère de l'art antérieur.

Dans ce procédé, la paroi séparatrice de la colonne de finition n'est pas jointive avec la partie haute de la colonne. L'acide acrylique de grade polymère est récupéré par soutirage latéral de l'autre côté de la section d'alimentation.

Avec cette configuration, l'injection de l'agent chimique est réalisée dans le flux d'alimentation de la colonne, ou dans la section d'alimentation de la colonne, obligatoirement à un niveau situé au-dessus du soutirage latéral. Le risque d'engendrer la formation de solides qui vont s'accumuler dans la colonne est important, et il n'est pas possible de placer un système de récupération des solides.

Le nombre d'étages de séparation résultant du fonctionnement de ce type de colonne ne permet pas d'éliminer complètement l'impureté protoanémonine.

En effet, la séparation de l'acide acrylique avec la protoanémonine va dépendre en grande partie du nombre d'étages de séparation disponibles. Dans le cas de la configuration représentée à la figure 4, la section 36 doit réaliser deux opérations : i) garantir la séparation des composés légers avec le produit du soutirage latéral, ce qui peut être réalisé schématiquement par les plateaux compris entre la partie supérieure et l'emplacement du soutirage latéral de la partie 36, ii) puis faire la séparation des composés lourds et de l'acide acrylique entre la partie inférieure et le soutirage latéral de la partie 36.

Dans le procédé selon l'invention, ces 2 opérations peuvent être respectivement réalisées par les sections 35 et 36 dans leur intégralité.

De plus, l'énergie liée au débit de recyclage en tête de cette colonne de finition vers la colonne de déshydratation pour recycler les composés légers et garantir une qualité constante de l'acide acrylique glacial extrait par soutirage latéral, reste un inconvénient dans ce procédé de l'art antérieur.

Le procédé selon l'invention permet de surmonter avantageusement tous ces inconvénients de l'art antérieur.

L'invention sera maintenant illustrée par les exemples suivants, qui n'ont pas pour but de limiter la portée de l'invention, définie par les revendications annexées.

### PARTIE EXPERIMENTALE

Dans les exemples, les pourcentages sont indiqués en poids pour les composés principaux sauf indication contraire, et les abréviations suivantes ont été utilisées :
H20 : eau
AA : acide acrylique
BZH : benzaldéhyde
ACOH : acide acétique
FURF : furfuraldéhyde
PTA : protoanémonine

Des simulations à l'aide du logiciel ASPEN^{®} ont été utilisées pour illustrer le procédé selon l'invention comparativement au procédé selon l'art antérieur décrit dans le brevet EP 2 066 613 B1.

### Exemple 1 (comparatif) : Purification à l'aide d'une colonne de finition classique avec soutirage latéral

Le procédé de récupération et purification d'acide acrylique produit par oxydation en 2 étapes du propylène tel que décrit dans le brevet EP 2 066 613B1 et représenté sur la Figure 1, fournit en soutirage latéral de la colonne de finition (17), un flux (5) d'acide acrylique purifié.

Le flux (3) de pied de la colonne de déshydratation (10) constitue l'alimentation au niveau du plateau supérieur de la colonne de finition (17). Cette colonne comporte un rebouilleur (18) en fond, un condenseur (19) en tête et un soutirage latéral en phase gaz (5) qui est ensuite condensé dans un échangeur (non représenté). La colonne de finition (17) comporte 17 étages théoriques, et le soutirage latéral est réalisé au niveau du plateau théorique 13 compté à partir du haut de la colonne. Une partie du flux de pied de colonne recirculé à travers le rebouilleur (18) est soutiré (flux 6), et après condensation dans l'échangeur (19), le flux gazeux (4) de tête de colonne est renvoyé sous forme liquide dans la boucle de recirculation (20) de pied de colonne de déshydratation,

Le tableau 1 précise les principales conditions opératoires de la colonne de finition (débit, température, et pression) et la composition des différents flux :

**Tableau 1**

| | | Alimentation | Produit purifié | Pied de colonne | Tête de colonne recyclée |
|---|---|---|---|---|---|
| Flux | | 3 | 5 | 6 | 4 |
| Composition | | | | | |
| | H2O | 4,43% | <0,01% | <0,001% | 5,86% |
| | ACOH | 10,05% | 0,05% | <0,001% | 13,29% |
| | AA | 85,01% | 99,81% | 84,18% | 80,62% |
| | FURF | 0,0112% | 0,0142% | 0,17% | 0,0052% |
| | BZH | 0,0154% | 0,0082% | 0,41% | 0,0049% |
| | PTA | 0,0090% | 0,0048% | 0,24% | 0,0028% |
| Débit (kg/hr) | | 49800 | 11000 | 1195 | 37642 |
| Température (°C) | | 62 | 96 | 102 | 74 |
| Pression (bar) | | 0,12 | 0,21 | 0,24 | 0,12 |

La qualité d'AA obtenue au soutirage latéral est supérieure à 99,8% et conforme à la qualité attendue d'un acide acrylique de grade "technique", pour la synthèse d'esters acryliques par exemple. Pour la synthèse de polymères même peu exigeants en termes de qualité (par exemple pour la synthèse de polymères de masses moléculaires intermédiaires), cette qualité n'est pas suffisante, en particulier à cause de la présence de teneurs trop élevées en aldéhydes (142 ppm de furfuraldéhyde et 82 ppm de benzaldéhyde) et en protoanémonine.

Ce procédé de récupération nécessite une consommation énergétique à fournir au bouilleur de la colonne de finition de 6,6 Gcal/h.

### Exemple 2 (suivant l'invention) : Purification à l'aide d'une colonne de finition à paroi séparatrice selon un mode de réalisation de l'invention

En référence à la Figure 2, la colonne de finition est équipée d'une paroi interne jointive avec le dôme supérieur, non jointive avec le fond, délimitant une section d'alimentation (35) et une section de soutirage (36) qui permet d'obtenir en tête la qualité purifiée d'acide acrylique. La section d'alimentation permet d'éliminer les composés légers recyclés en pied de colonne de déshydratation après condensation dans un échangeur (40).

Dans cette configuration, les sections d'alimentation (35) et de soutirage (36) comportent chacune 14 plateaux théoriques et l'espace inférieur de soutirage de pied comporte 3 étages théoriques.

La distillation des mélanges liquide / gaz transitant dans chacune des sections est assurée par un échangeur commun (rebouilleur 18) en fond de colonne. La colonne de finition est alimentée par le flux de fond de colonne de déshydratation (10) au niveau du plateau supérieur de la section d'alimentation (35). Le flux d'acide acrylique purifié (5) est soutiré en tête de section de soutirage (36), après condensation du mélange gazeux dans un échangeur (19), et en partie renvoyé sous forme de reflux (8) en tête de section (36).

La colonne à paroi séparatrice est alimentée par un flux d'alimentation (3) inchangé en débit et composition par rapport à celui de l'exemple 1. De même, les débits des flux de légers recyclés vers la colonne de déshydratation (4), de lourds éliminés en pied de colonne (6) et de produit purifié (5) sont inchangés par rapport à ceux de l'exemple 1.

Le tableau 2 précise les conditions opératoires de la colonne de finition (débit, température, et pression) et la composition des différents flux :

**Tableau 2**

| | | Alimentation | Produit purifié | Pied de colonne | Tête de colonne recyclée |
|---|---|---|---|---|---|
| Flux | | 3 | 5 | 6 | 4 |
| Composition | | | | | |
| | H2O | 4,40% | <0,01% | <0,001% | 5,90% |
| | ACOH | 10,10% | 0,0119% | <0,001% | 13,30% |
| | AA | 85,10% | >99,95% | 89,10% | 80,70% |
| | FURF | 0,0112% | 0,0004% | 0,40% | <0,0001% |
| | BZH | 0,0154% | <0,0001% | 0,50% | <0,0001% |
| | PTA | 0,0090% | <0,0001% | 0,29% | <0,0001% |
| Débit (kg/hr) | | 49800 | 11000 | 1195 | 37642 |
| Température (°C) | | 62 | 82 | 90 | 74 |
| Pression (bar) | | 0,12 | 0,12 | 0,15 | 0,12 |

Selon l'invention, l'acide acrylique technique soutiré en tête de la section de soutirage de la colonne à paroi séparatrice a une qualité améliorée par rapport à celle obtenue dans l'exemple 1, en particulier pour les teneurs en benzaldéhyde, furfural et protoanémonine.

Sans être suffisante pour la synthèse de polymères mettant en oeuvre une réaction particulièrement sensible à la présence d'impuretés, en particulier d'aldéhydes (par exemple pour la synthèse de polymères de hautes masses moléculaires), cette qualité d'acide acrylique technique améliorée permet néanmoins d'être utilisée pour certaines applications moins sensibles.

### Exemple 3 (suivant l'invention) : Purification à l'aide d'une colonne de finition à paroi séparatrice selon un mode de réalisation de l'invention

On a reproduit l'exemple 2, avec des conditions d'alimentation identiques, mais avec une colonne à paroi séparatrice comportant une section d'alimentation (35) et une section de soutirage (36) comportant chacune 17 plateaux théoriques et une section d'espace inférieur de soutirage du pied comportant 3 étages théoriques.

Le tableau 3 décrit les paramètres de fonctionnement et les compositions des flux.

**Tableau 3**

| | | Alimentation | Produit purifié | Pied de colonne | Tête de colonne recyclée |
|---|---|---|---|---|---|
| Flux | | 3 | 5 | 6 | 4 |
| Composition | | | | | |
| | H2O | 4,40% | <0,01% | <0,001% | 5,90% |
| | ACOH | 10,10% | <0,01% | <0,001% | 13,30% |
| | AA | 85,10% | >99,95% | 89,10% | 80,70% |
| | FURF | 0,0112% | <0,0001% | 0,50% | 0,0008% |
| | BZH | 0,0154% | <0,0001% | 0.,0% | 0,0008% |
| | PTA | 0,0090% | <0,0001% | 0,29% | 0,0005% |
| Débit (kg/hr) | | 49800 | 11000 | 1158 | 37642 |
| Température (°C) | | 62 | 82 | 92 | 74 |
| Pression (bar) | | 0,12 | 0,12 | 0,16 | 0,12 |

Dans cette configuration où chacune des sections de la colonne de finition comporte un nombre de plateaux plus élevé, l'acide acrylique soutiré en tête de la section de soutirage présente une pureté supérieure à celle obtenue dans l'exemple 2, et répond aux exigences de l'acide acrylique glacial (ou grade polymère). Cette qualité d'acide acrylique peut être utilisée directement pour la synthèse des polymères les plus techniques, sans avoir recours à un agent de traitement chimique visant à réduire la teneur en aldéhydes résiduels, ou sans nécessiter de traitement complémentaire, par exemple par cristallisation ou par distillation.

Dans cet exemple, la consommation énergétique à fournir au bouilleur de la colonne de finition, est de 7,7 GCal/h.

### Exemple 4 (comparatif) : Récupération d'acide acrylique glacial (de grade polymère)

Par simulation à l'aide du logiciel ASPEN^{®}, l'acide acrylique technique obtenu en soutirage latéral selon le procédé de l'art antérieur de l'exemple 1 doit être purifié dans une colonne supplémentaire comportant 19 étages théoriques pour obtenir la même qualité d'acide acrylique glacial obtenu dans l'exemple 3 selon le procédé de l'invention.

Le tableau 4 indique la composition des flux séparés en tête et en pied de la colonne supplémentaire.

**Tableau 4**

| Flux | | Alimentation | Produit purifié | Pied de colonne |
|---|---|---|---|---|
| Composition | | Flux 5 de l'exemple 1 | | |
| | ACOH | 0,05% | 0,0608% | 0,0013% |
| | AA | 99,80% | 99,90% | 99,90% |
| | FURF | 0,0143% | <0,0001% | 0,0783% |
| | BZH | 0,0082% | <0,0001% | 0,0453% |
| | PTA | 0,0048% | <0,0001% | 0,05% |
| Débit (kg/hr) | | 11000 | 9000 | 2000 |

Pour cette purification complémentaire, l'énergie à mettre en oeuvre est de 5,5 Gcal/h.

Le procédé de l'art antérieur, combinant une colonne de finition classique avec soutirage latéral d'un flux d'acide acrylique technique et une colonne de distillation complémentaire (résultant en la mise en oeuvre de 17+19, soit 36 étages théoriques) nécessite la fourniture énergétique au total de 6,6 + 5,5 Gcal/h, soit 12,1 Gcal/h, donc nettement supérieure à l'énergie de 7,7 Gcal/h mise en oeuvre dans l'exemple 3, à qualité d'acide acrylique glacial équivalente.

Le procédé selon l'invention permet de réduire de 35% la consommation énergétique pour obtenir une qualité identique d'acide acrylique glacial.

La colonne à paroi séparatrice nécessite moins d'étages théoriques (20 plateaux de part et d'autre de la paroi) pour obtenir la même qualité d'acide acrylique glacial. Il en résulte un coût d'investissement plus faible lié à une hauteur limitée de la colonne de finition.

Cet avantage est encore accentué lors de la mise en oeuvre de plateaux perforés sans déversoir de type "Dual Flow " qui sont généralement utilisés pour la distillation de produits particulièrement thermosensibles comme l'acide acrylique (risques de polymérisation fortement augmentés lorsque la température est plus élevée).

## Revendications

1. Procédé de récupération d'acide (méth)acrylique purifié, en l'absence de solvant organique, à partir d'un mélange réactionnel gazeux comprenant de l'acide (méth)acrylique obtenu par oxydation en phase gazeuse d'un précurseur de l'acide (méth)acrylique, comprenant au moins les étapes suivantes :
a) le mélange réactionnel gazeux est soumis à une déshydratation sans utiliser de solvant azéotropique dans une première colonne de distillation dite colonne de déshydratation, conduisant à un flux de tête dont une partie au moins est condensée et renvoyée à la colonne de déshydratation sous forme de reflux, et à un flux de pied dont une partie au moins est renvoyée en reflux dans la partie inférieure de la colonne de déshydratation pour former une boucle de recirculation ;
b) le flux de pied de la colonne de déshydratation est envoyé au moins en partie dans une seconde colonne de distillation dite colonne de finition, permettant de séparer un flux de pied contenant des composés lourds, et un flux de tête contenant des composés légers, dont une partie au moins est renvoyée dans la colonne de déshydratation ;
ledit procédé étant **caractérisé en ce que** :
i) la colonne de finition est équipée d'une paroi séparatrice, la paroi étant jointive avec le dôme supérieur de la colonne en partie haute et non jointive avec le fond de la colonne en partie basse, séparant ainsi la colonne en deux sections équipées d'éléments internes de distillation assurant le contact gaz-liquide, dont l'espace inférieur communique avec l'espace de fond de colonne, et dont l'espace de tête est séparé en deux zones hermétiques, l'alimentation de ladite colonne s'effectuant d'un seul côté de la paroi séparatrice, et
ii) un flux gazeux riche en composés légers et comprenant de l'eau est extrait en tête de la section d'alimentation, puis recyclé, après condensation, au moins en partie dans la boucle de recirculation en pied de la colonne de déshydratation, et
iii) un flux gazeux d'acide (méth)acrylique purifié extrait sous forme gazeuse de la colonne de finition en tête de la section située de l'autre côté de la section d'alimentation, est soutiré après condensation, une partie du flux condensé étant renvoyée comme reflux liquide en tête de la section de soutirage.

2. Procédé selon la revendication 1 **caractérisé en ce que** le précurseur de l'acide (méth)acrylique est l'acroléine, obtenue par oxydation de propylène ou par oxydéshydrogénation de propane.

3. Procédé selon la revendication 1 **caractérisé en ce que** le précurseur de l'acide (méth)acrylique est la méthacroléine obtenue par oxydation d'isobutylène et/ou de tert-butanol ou à partir d'oxydéshydrogénation de butane et/ou isobutane.

4. Procédé selon la revendication 1 **caractérisé en ce que** le précurseur de l'acide (méth)acrylique comprend du carbone d'origine renouvelable.

5. Procédé selon l'une quelconque des revendications précédentes **caractérisé en ce que** l'alimentation de la colonne de finition s'effectue en tête au niveau du plateau supérieur de la section d'alimentation, et de manière optionnelle, une partie du flux gazeux qui est extrait en tête de la section d'alimentation, est renvoyée après condensation dans le flux d'alimentation de la colonne de finition.

6. Procédé selon l'une quelconque des revendications 1 à 4 **caractérisé en ce que** l'alimentation de la colonne de finition s'effectue en un point situé plus bas que le plateau supérieur de la section d'alimentation, et une partie du flux gazeux qui est extrait en tête de la section d'alimentation, est renvoyée après condensation comme reflux liquide en tête de la section d'alimentation.

7. Procédé selon l'une quelconque des revendications précédentes **caractérisé en ce que** la section d'alimentation de la colonne de finition comporte un nombre de plateaux théoriques compris entre 5 et 20, de préférence entre 15 et 20.

8. Procédé selon l'une quelconque des revendications précédentes **caractérisé en ce que** la section de soutirage de la colonne de finition comporte un nombre de plateaux théoriques compris entre 2 et 20, de préférence entre 15 et 20.

9. Procédé selon l'une quelconque des revendications précédentes **caractérisé en ce que** l'espace inférieur de la colonne de finition situé sous la paroi séparatrice est équipé d'éléments de rectification représentant entre 1 et 5 plateaux théoriques

10. Procédé selon l'une quelconque des revendications précédentes **caractérisé en ce qu'**il comprend en outre l'introduction dans la colonne de finition, d'un agent de traitement chimique visant à réduire la teneur en aldéhydes résiduels.

11. Procédé selon la revendication 10 **caractérisé en ce que** l'agent de traitement chimique des aldéhydes est choisi parmi :
• des amines, telles que par exemple, sans limitation, la monoéthanolamine, l'éthylène diamine la glycine, la diéthylènetriamine, la dipropylènetriamine, l'ortho-, la para-, et la meta-phenylenediamine.,
• des composés de la famille de l'aniline, comme par exemple, sans limitation, l'aniline, l'ortho-, la para-, et la meta-méthylaniline
• des composés de la famille des hydrazines, tel que, sans limitation, l'hydrazine et ses sels, l'hydrate d'hydrazine, le sulfate d'hydrazine, les carboxylates d'hydrazine, le chlorhydrate d'hydrazine, la phénylhydrazine, la 4-nitrophénylhydrazine, et la 2,4-dinitrophénylhydrazine, ou encore l'aminoguanidine et ses sels, comme l'hydrogénocarbonate d'aminoguanidine.
• Des composés de la famille des hydrazides, comme par exemple, sans limitation, les hydrazides d'acides carboxyliques et leurs sels, tels que les hydrazides d'acides formique, acétique, propionique, butanoïque, pentanoïque, maléique et les dihydrazides d'acides adipique et succinique, l'urée ou des dérivés de l'urée et de l'hydrazine, comme le semicarbazide ou le carbohydrazide et leurs sels ;
seuls ou leurs mélanges en toutes proportions.

12. Procédé selon la revendication 10 ou 11 **caractérisé en ce que** l'agent de traitement chimique est introduit dans le flux d'alimentation de la colonne de finition, de préférence par l'intermédiaire d'un dispositif de mélange comprenant au moins une capacité assurant la dispersion efficace de l'agent chimique avec le flux d'alimentation.

13. Procédé selon la revendication 10 ou 11 **caractérisé en ce que** l'agent de traitement chimique est introduit directement dans la colonne de finition en un point situé entre la tête et le pied de colonne, plus bas que le plateau où est effectuée l'alimentation de la colonne, de préférence en un point situé entre environ un tiers et deux tiers de la hauteur de la section d'alimentation de la colonne de finition.

14. Procédé selon la revendication 10 ou 11 **caractérisé en ce qu'**un soutirage latéral d'un flux gazeux comprenant de l'acide (méth)acrylique de qualité intermédiaire est effectué à partir de la section de soutirage.

15. Procédé selon l'une quelconque des revendications 1 à 9 comprenant en outre un traitement complémentaire de l'acide (méth)acrylique purifié par cristallisation fractionnée, ou par distillation éventuellement en présence d'un composé réagissant avec les aldéhydes résiduels, conduisant à une qualité d'acide (méth)acrylique de grade polymère.

16. Procédé de production d'acide (méth)acrylique purifié comprenant au moins les étapes suivantes :
A) on soumet à une oxydation en phase gazeuse au moins un précurseur d'acide (méth)acrylique pour former un mélange réactionnel gazeux comprenant de l'acide (méth)acrylique ;
B) on refroidit le mélange réactionnel gazeux ;
C) on soumet le mélange réactionnel gazeux refroidi au procédé de récupération de l'acide (méth)acrylique tel que défini selon l'une quelconque des revendications précédentes.

## Patentansprüche

1. Verfahren zur Rückgewinnung von gereinigter Methacrylsäure ohne organisches Lösungsmittel aus einem gasförmigen Reaktionsgemisch, das Methacrylsäure enthält, die durch Gasphasenoxidation einer Methacrylsäurevorstufe erhalten wird, umfassend mindestens folgende Schritte:
a) das gasförmige Reaktionsgemisch wird ohne Einsatz eines azeotropen Lösungsmittels in einer ersten als Dehydrierungskolonne bezeichneten Destillationskolonne dehydriert, wodurch ein Strom am Kopf, von dem zumindest ein Teil kondensiert und als Rücklauf zur Dehydrierungskolonne zurückgeleitet wird, und ein Sumpfstrom entsteht, von dem zumindest ein Teil als Rücklauf in den unteren Teil der Dehydrierungskolonne zurückgeleitet wird und so ein Rücklaufkreislauf entsteht;
b) der Sumpfstrom der Dehydrierungskolonne wird zumindest teilweise in eine zweite als Abschlusskolonne bezeichnete Destillationskolonne geleitet, in der sich ein Sumpfstrom, der schwer flüchtige Bestandteile enthält, und ein Kopfstrom, der leicht flüchtige Bestandteile enthält und von dem zumindest ein Teil in die Dehydrierungskolonne zurückgeleitet wird, abtrennen lassen;
wobei das Verfahren **dadurch gekennzeichnet ist, dass**:
i) die Abschlusskolonne mit einer Trennwand versehen ist, wobei die Wand mit der oberen Haube der Kolonne im oberen Abschnitt verbunden ist und nicht mit dem Boden der Kolonne im unteren Abschnitt verbunden ist, wodurch die Kolonne in zwei Teilabschnitte unterteilt ist, die mit inneren Destillationselementen versehen ist, die für den Gas-Flüssigkeit-Kontakt sorgen, wobei der untere Raum mit dem Kolonnenbodenraum in Verbindung steht und der Kopfraum in zwei hermetisch abgedichtete Bereiche unterteilt ist, wobei der Zulauf in die Kolonne von einer einzigen Seite der Trennwand aus erfolgt, und
ii) ein gasförmiger Strom mit einem hohen Gehalt an leicht flüchtigen Bestandteilen, der Wasser enthält, am Kopf des Zulaufabschnitts abgezogen, dann nach der Kondensation zumindest teilweise wieder in den Rücklaufkreislauf am Sumpf der Dehydrierungskolonne zurückgeführt wird, und
iii) ein gasförmiger gereinigter Methacrylsäurestrom, der gasförmig aus der Abschlusskolonne am Kopf des Abschnitts, der sich auf der anderen Seite des Zulaufabschnitts befindet, nach der Kondensation abgezogen wird, wobei ein Teil des kondensierten Stroms als flüssiger Rücklauf am Kopf des Abzugsabschnitts zurückgeleitet wird.

2. Verfahren nach Anspruch 1, **dadurch gekennzeichnet, dass** die Methacrylsäurevorstufe Acrolein ist, das durch Oxidation von Propen oder durch oxydative Dehydrierung von Propan erhalten wird.

3. Verfahren nach Anspruch 1, **dadurch gekennzeichnet, dass** die Methacrylsäurevorstufe Methacrolein ist, das durch Oxidation von Isobutylen und/oder tert-Butanol oder aus der oxydativen Dehydrierung von Butan und/oder Isobutan erhalten wird.

4. Verfahren nach Anspruch 1, **dadurch gekennzeichnet, dass** die Methacrylsäurevorstufe erneuerbaren Kohlenstoff umfasst.

5. Verfahren nach einem der vorhergehenden Ansprüche, **dadurch gekennzeichnet, dass** der Zulauf in die Abschlusskolonne am Kopf im Bereich des oberen Bodens des Zulaufabschnitts erfolgt und gegebenenfalls ein Teil des Gasstroms, der am Kopf des Zulaufabschnitts abgezogen wird, nach der Kondensation in den Zulaufstrom der Abschlusskolonne zurückgeleitet wird.

6. Verfahren nach einem der Ansprüche 1 bis 4, **dadurch gekennzeichnet, dass** der Zulauf in die Abschlusskolonne an einer Stelle erfolgt, die unterhalb des oberen Bodens des Zulaufabschnitts liegt, und ein Teil des Gasstroms, der am Kopf des Zulaufabschnitts abgezogen wird, nach der Kondensation als flüssiger Rücklauf am Kopf des Zulaufabschnitts zurückgeleitet wird.

7. Verfahren nach einem der vorhergehenden Ansprüche, **dadurch gekennzeichnet, dass** der Zulaufabschnitt der Abschlusskolonne eine Anzahl theoretischer Böden zwischen 5 und 20, vorzugsweise zwischen 15 und 20 aufweist.

8. Verfahren nach einem der vorhergehenden Ansprüche, **dadurch gekennzeichnet, dass** der Abzugsabschnitt der Abschlusskolonne eine Anzahl theoretischer Böden zwischen 2 und 20, vorzugsweise zwischen 15 und 20 aufweist.

9. Verfahren nach einem der vorhergehenden Ansprüche, **dadurch gekennzeichnet, dass** der untere Raum der Abschlusskolonne, der unter der Trennwand liegt, mit Rektifikationselementen versehen ist, die zwischen 1 und 5 theoretische Böden darstellen.

10. Verfahren nach einem der vorhergehenden Ansprüche, **dadurch gekennzeichnet, dass** es ferner das Einleiten eines Mittels zur chemischen Behandlung, mit dem der Restaldehydgehalt gesenkt werden soll, in die Abschlusskolonne umfasst.

11. Verfahren nach Anspruch 10, **dadurch gekennzeichnet, dass** das Mittel zur chemischen Behandlung der Aldehyde ausgewählt wird aus:
• Aminen, beispielsweise Monoethanolamin, Ethylendiamin, Glycin, Diethylentriamin, Dipropylentriamin, ortho-, para- und meta-Phenylendiamin, ohne darauf beschränkt zu sein,
• Verbindungen aus der Gruppe der Aniline wie zum Beispiel Anilin, ortho-, para- und meta-Methylanilin, ohne darauf beschränkt zu sein,
• Verbindungen aus der Gruppe der Hydrazine wie zum Beispiel Hydrazin und seine Salze, Hydrazinhydrat, Hydrazinsulfat, Hydrazincarboxylate, Hydrazinchlorhydrat, Phenylhydrazin, 4-Nitrophenylhydrazin und 2,4-Dinitrophenylhydrazin oder auch Aminoguanidin und seine Salze wie Aminoguanidinhydrogencarbonat, ohne darauf beschränkt zu sein,
• Verbindungen aus der Gruppe der Hydrazide wie zum Beispiel Carbonsäurehydrazide und ihre Salze, beispielsweise Methan-, Ethan-, Propan-, Butan-, Pentan-, Maleinsäurehydrazide und Adipin- und Bernsteinsäuredihydrazide, Harnstoff oder Harnstoff- und Hydrazinderivate, beispielsweise Semicarbazid oder Carbodihydrazid und seine Salze, ohne darauf beschränkt zu sein;
allein oder Mischungen derselben in sämtlichen Anteilen.

12. Verfahren nach Anspruch 10 oder 11, **dadurch gekennzeichnet, dass** das Mittel zur chemischen Behandlung vorzugsweise über eine Mischvorrichtung, die zumindest über eine Leistung verfügt, mit der die wirksame Verteilung des chemischen Mittels im Zulauf gewährleistet ist, in den Zulauf der Abschlusskolonne eingeleitet wird.

13. Verfahren nach Anspruch 10 oder 11, **dadurch gekennzeichnet, dass** das Mittel zur chemischen Behandlung unmittelbar in die Abschlusskolonne eingeleitet wird, an einer Stelle, die zwischen Kolonnenkopf und -sumpf liegt, unterhalb des Bodens, an dem der Zulauf in die Kolonne erfolgt, vorzugsweise an einer Stelle, die zwischen ungefähr einem Drittel und zwei Dritteln der Höhe des Zulaufabschnitts der Abschlusskolonne liegt.

14. Verfahren nach Anspruch 10 oder 11, **dadurch gekennzeichnet, dass** vom Abzugsabschnitt ein seitlicher Abzug eines Gasstroms erfolgt, der Methacrylsäure mittlerer Qualität umfasst.

15. Verfahren nach einem der Ansprüche 1 bis 9, das ferner eine ergänzende Behandlung der gereinigten Methacrylsäure durch fraktionierte Kristallisation oder durch eine Destillation, gegebenenfalls in Gegenwart einer Verbindung, die mit den Restaldehyden reagiert, umfasst, die zu einer Methacrylsäurereinheit "polymer grade" führt.

16. Verfahren zur Herstellung von gereinigter Methacrylsäure, das mindestens die folgenden Schritte umfasst:
A) Durchführen einer Gasphasenoxidation mit mindestens einer Methacrylsäurevorstufe zum Bilden eines gasförmigen Reaktionsgemischs, das Methacrylsäure enthält;
B) Abkühlen des gasförmigen Reaktionsgemischs;
C) Durchführen des Verfahrens zur Rückgewinnung von Methacrylsäure nach einem der vorhergehenden Ansprüche mit dem abgekühlten gasförmigen Reaktionsgemisch.

## Claims

1. A method for recovering purified (meth)acrylic acid, in the absence of organic solvent, from a gaseous reaction mixture comprising (meth)acrylic acid obtained by gaseous phase oxidation of a (meth)acrylic acid precursor, comprising at least the following steps:
a) the gaseous reaction mixture is subjected to a dehydration without using an azeotropic solvent in a first distillation column called a dehydration column, leading to a flow from the top of which at least part is condensed and sent back to the dehydration column in the form of reflux, and to a flow from the bottom of which at least part is sent back as reflux into the lower part of the dehydration column to form a recirculation loop;
b) the flow from the bottom of the dehydration column is sent at least in part into a second distillation column called a finishing column, which can separate a flow from the bottom containing heavy compounds, and a flow from the top containing light compounds, of which at least part is sent back to the dehydration column;
said method being **characterized in that**:
i) the finishing column is equipped with a separating wall, the wall being connected with the upper done of the column in the top portion and not connected with the bottom of the column in the lower portion, thereby separating the column into two sections equipped with internal distillation elements that ensure gas-liquid contact, of which the lower space communicates with the space at the bottom of the column, and of which the headspace is separated into two sealed areas, said column being fed from a single side of the separating wall, and
ii) a gaseous flow rich in light compounds and comprising water is extracted at the top of the feed section, then recycled, after condensation, at least in part in the recirculation loop at the bottom of the dehydration column, and
iii) a flow of purified (meth)acrylic acid extracted in gaseous form from the finishing column at the top of the section located on the other side of the feed section, is drawn off after condensation, part of the condensed flow being sent back as liquid reflux at the top of the draw-off section.

2. The method according to claim 1 **characterized in that** the (meth)acrylic acid precursor is acrolein, obtained by the oxidation of propylene or by the oxydehydrogenation of propane.

3. The method according to claim 1 **characterized in that** the (meth)acrylic acid precursor is methacrolein obtained by the oxidation of isobutylene and/or of tert-butanol or from the oxydehydrogenation of butane and/or isobutane.

4. The method according to claim 1 **characterized in that** the (meth)acrylic acid precursor comprises carbon from a renewable source.

5. The method according to any one of the previous claims **characterized in that** the finishing column is fed at the top at the upper tray of the feed section, and optionally, a portion of the gaseous flow that is extracted at the top of the feed section, is sent back after condensation into the feed flow of the finishing column.

6. The method according to any one of claims 1 to 4 **characterized in that** the finishing column is fed at a point located lower than the upper tray of the feed section, and a portion of the gaseous flow that is extracted at the top of the feed section, is sent back after condensation as liquid reflux at the top of the feed section.

7. The method according to any one of the previous claims **characterized in that** the feed section of the finishing column includes a number of theoretical trays inclusively between 5 and 20, preferably between 15 and 20.

8. The method according to any one of the previous claims **characterized in that** the draw-off section of the finishing column includes a number of theoretical trays inclusively between 2 and 20, preferably between 15 and 20.

9. The method according to any one of the previous claims **characterized in that** the lower space of the finishing column located under the separating wall is equipped with rectification elements representing between 1 and 5 theoretical trays

10. The method according to any one of the previous claims **characterized in that** it further comprises the introduction into the finishing column, of a chemical processing agent aiming to reduce the residual aldehyde content.

11. The method according to claim 10 **characterized in that** the chemical processing agent for aldehydes is chosen from:
• amines, such as for example, without limitation, monoethanolamine, ethylene diamine, glycine, diethylenetriamine, dipropylenetriamine, ortho-, para-, and meta-phenylenediamine.,
• compounds in the aniline family, such as for example, without limitation, aniline, ortho-, para-, and meta-methylaniline
• compounds in the hydrazine family, such as, without limitation, hydrazine and its salts, hydrazine hydrate, hydrazine sulfate, hydrazine carboxylates, hydrazine hydrochloride, phenylhydrazine, 4-nitrophenylhydrazine, and 2,4-dinitrophenylhydrazine, or also aminoguanidine and its salts, like aminoguanidine hydrogen carbonate.
• compounds in the hydrazide family, such as for example, without limitation, carboxylic acid hydrazides and their salts, such as the hydrazides of formic, acetic, propionic, butanoic, pentanoic, maleic acids and the dihydrazides of adipic and succinic acids, urea or urea and hydrazine derivatives, such as semicarbazide or carbohydrazide and their salts;
alone or their mixtures in any proportions.

12. The method according to claim 10 or 11 **characterized in that** the chemical processing agent is introduced into the feed flow of the finishing column, preferably by means of a mixing device comprising at least a capacity ensuring the effective dispersion of the chemical agent with the feed flow.

13. The method according to claim 10 or 11 **characterized in that** the chemical processing agent is introduced directly into the finishing column at a point located between the top and the bottom of the column, lower than the tray where the column is fed, preferably at a point located between about one third and two thirds of the height of the feed section of the finishing column.

14. The method according to claim 10 or 11 **characterized in that** a side draw of a gaseous flow comprising (meth)acrylic acid with intermediate quality is made from the draw-off section.

15. The method according to any one of claims 1 to 9 further comprising an extra processing of purified (meth)acrylic acid by fractionated crystallization, or by distillation optionally in the presence of a compound reacting with the residual aldehydes, leading to a polymer-grade (meth)acrylic acid quality.

16. A method for producing purified (meth)acrylic acid comprising at least the following steps:
A) at least one (meth)acrylic acid precursor is subjected to an oxidation in gaseous phase to form a gaseous reaction mixture comprising (meth)acrylic acid;
B) the gaseous reaction mixture is cooled;
C) the cooled gaseous reaction mixture is subjected to the (meth)acrylic acid recovery method as defined according to any one of the previous claims.
